# EUROPEAN PATENT SPECIFICATION

(11) **EP 3 517 085 B1**
(45) Date of publication and mention of the grant of the patent: **09.09.2020**
(21) Application number: 18153585.7
(22) Date of filing: 26.01.2018
(51) Int. Cl.: A61F 13/15, B65D 85/07, B65B 63/02

(54) **HALTING ASSEMBLY FOR HYGIENIC AND SANITARY PRODUCTS**
HALTEVORRICHTUNG FÜR HYGIENE- UND SANITÄRPRODUKTE
ENSEMBLE D'ARRÊT POUR PRODUITS HYGIÉNIQUES ET SANITAIRES

(43) Date of publication of application: 31.07.2019
(73) Proprietor: Bucci Automations S.p.A., 48018 Faenza (RA) (IT)
(72) Inventor: ACCORDI, Marco, 48018 FAENZA RA (IT); CUSUMANO, Dario, 48018 FAENZA RA (IT); GUIDI, Manuel, 47023 CESENA FC (IT)
(74) Representative: Modiano, Micaela Nadia

(56) References cited:
- CN-B- 103 482 153
- CN-U- 201 472 694

## Description

The present invention relates to a halting assembly for hygienic and sanitary products, particularly suitable for packaging and packing lines in order to prepare the product in a configuration that ensures subsequent handy insertion within appropriate packaging.

Production and packaging lines generally comprise a first product production stage, a station for grouping and sorting them, and an actual terminal packaging unit.

In particular, with reference to products of the soft type, such as sanitary napkins, diapers and the like (therefore products which are particularly soft and include a lot of air), during grouping and sorting compaction is also necessary in order to be able to package them so as to reduce their bulk. This bulk reduction must in any case be preset as a function of the characteristics of the materials that constitute the products, in order to avoid the forming of permanent deformations of said products.

In order to provide the sorting, grouping and compaction of the products, stations are known which are constituted by an input line which is suitable for conveying products on a rack.

By means of an appropriate pickup element, the products are transferred from the rack to a compacting device in groups having predefined dimensions (which depend on the dimensions imposed by the final packaging).

The products, inside the rack, can be gathered within specific sliders or drawers designed for the accommodation of at least one product at a time (there are racks with drawers designed for the accommodation of a single product and racks with drawers designed for the accommodation of a predefined number of products arranged side by side).

In any case, the entry of the product in the drawer can also occur in an irregular manner: in particular, the product may arrive with excessive speed, proceeding by inertia beyond the terminal limit of the drawer itself (thus protruding from the drawer).

This condition is absolutely to be avoided, since the incorrect alignment, due to the transfer of a group of products to the machine designed for packing, may compromise the possibility to perform correct packaging of the group.

It is known to resort to stroke limiting shoulders on the bottom of the drawers, but these might cause further problems, since the product, by striking them, might be deformed (with consequent aesthetic defects and, in some cases, also functional ones) or might rebound, with the possibility of hindering the insertion of the subsequent products (going back upstream and assuming therefore an incorrect configuration for the subsequent packaging of the group of products that comprises it).

The aim of the present invention is to solve the problems described above, proposing a halting assembly for hygienic and sanitary products that ensures the halting of the products, within a respective drawer of a specific rack, in a predefined neighborhood of the ideal position.

Within this aim, an object of the invention is to propose a halting assembly for hygienic and sanitary products that does not generate any deformation of the products that enter respective drawers of a rack.

Another object of the invention is to propose a halting assembly for hygienic and sanitary products that prevents the product from rebounding backwards once it has reached the back wall of the respective drawer of a rack.

A further object of the present invention is to provide a halting assembly for hygienic and sanitary products that has modest costs, is relatively simple to provide in practice and is safe in application.

This aim, as well as these and other objects which will become better apparent hereinafter, are achieved by a halting assembly for hygienic and sanitary products of the type suitable for installation in a station for grouping products within adapted seats, said products arriving in sequence at said station, one after the other, from a supply apparatus arranged upstream, characterized in that it comprises at least one baffle that faces and is proximate to the terminal edge of the walls that delimit said seats and is actuated by at least one respective actuator controlled by a control and management unit, said baffle being movable between a first configuration which is external to said seat, in which it does not interfere with the at least one product in input thereto, and a second configuration for maximum overlap on said seat, in which it locks the product during first entry in said seat with a halting surface thereof.

Further characteristics and advantages of the invention will become better apparent from the description of a preferred but not exclusive embodiment of the halting assembly for hygienic and sanitary products according to the invention, illustrated by way of nonlimiting example in the accompanying drawings, wherein:

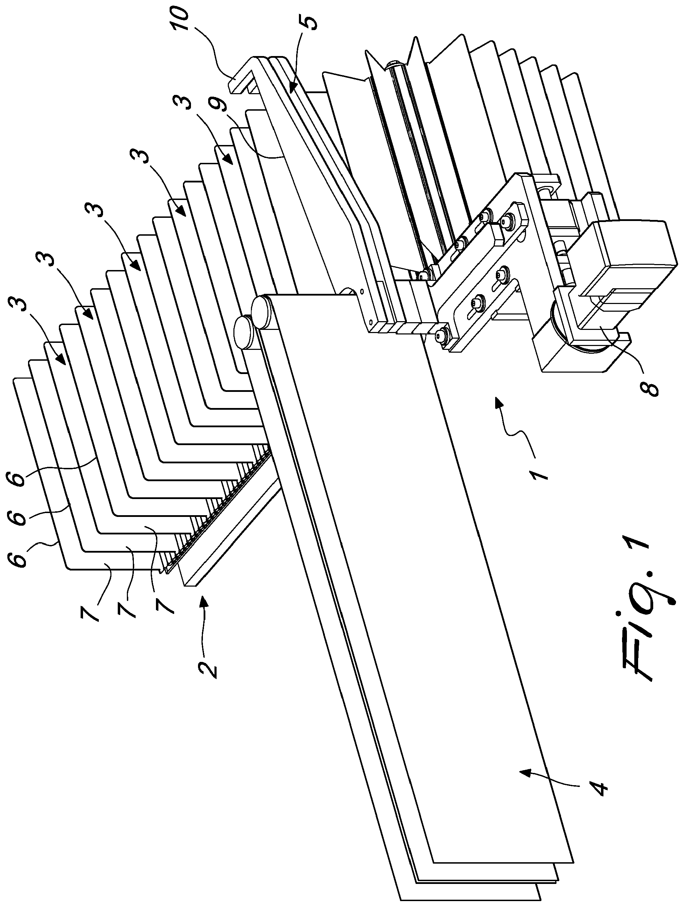

Figure 1 is a schematic perspective view of a halting assembly for hygienic and sanitary products according to the invention;

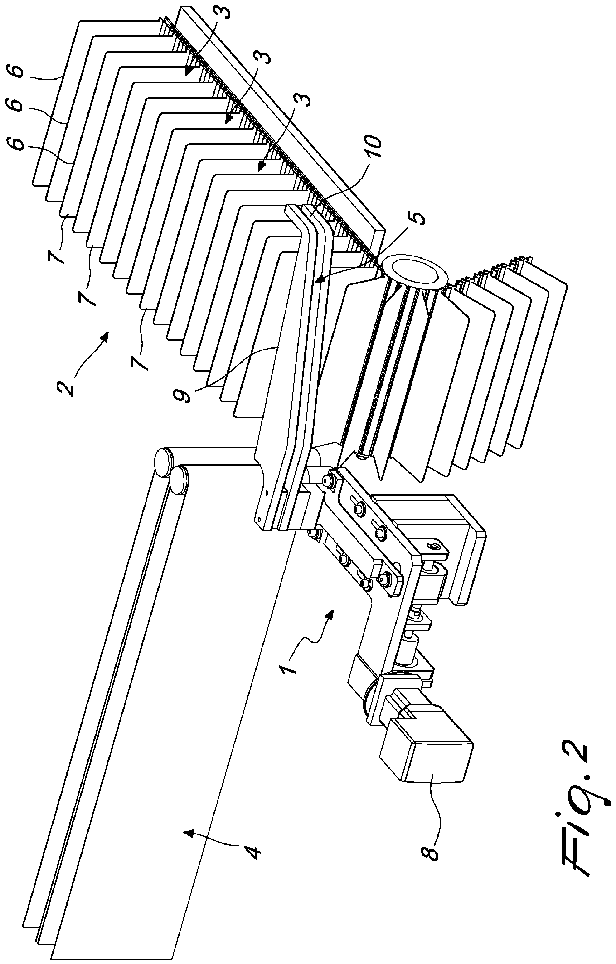

Figure 2 is a schematic perspective view, taken from a different viewpoint, of the assembly of Figure 1;

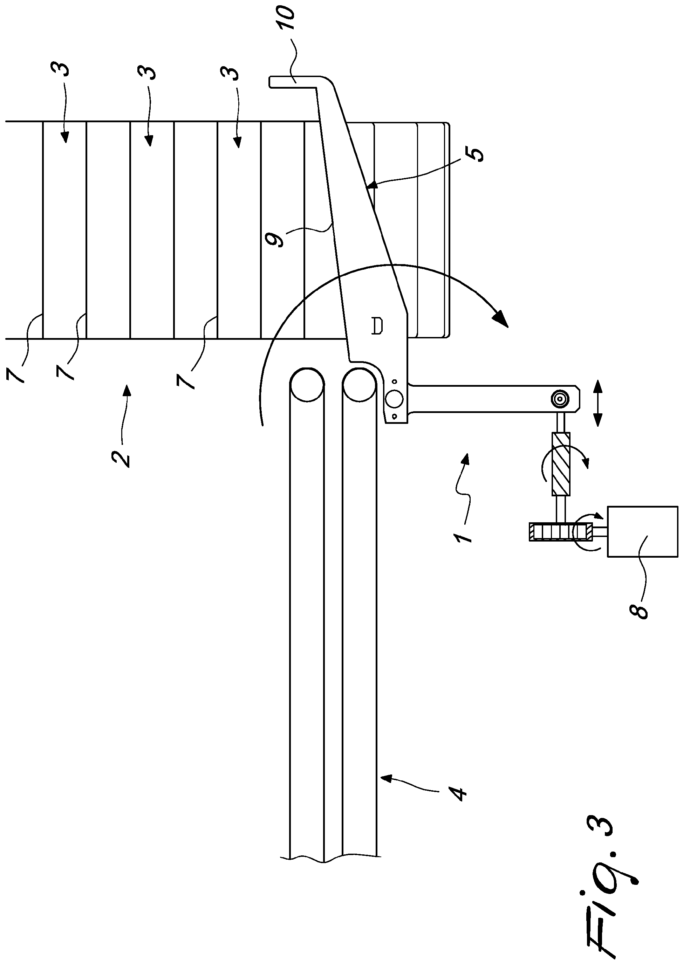

Figure 3 is a schematic top view of the assembly of Figure 1;

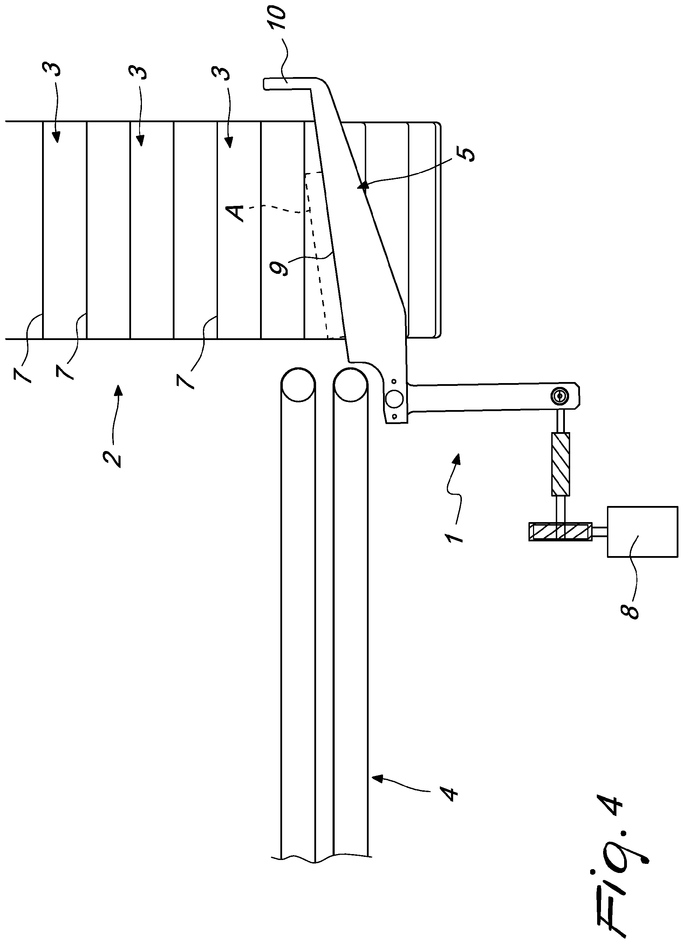

Figure 4 is a schematic top view of the assembly of Figure 1, showing a small product;

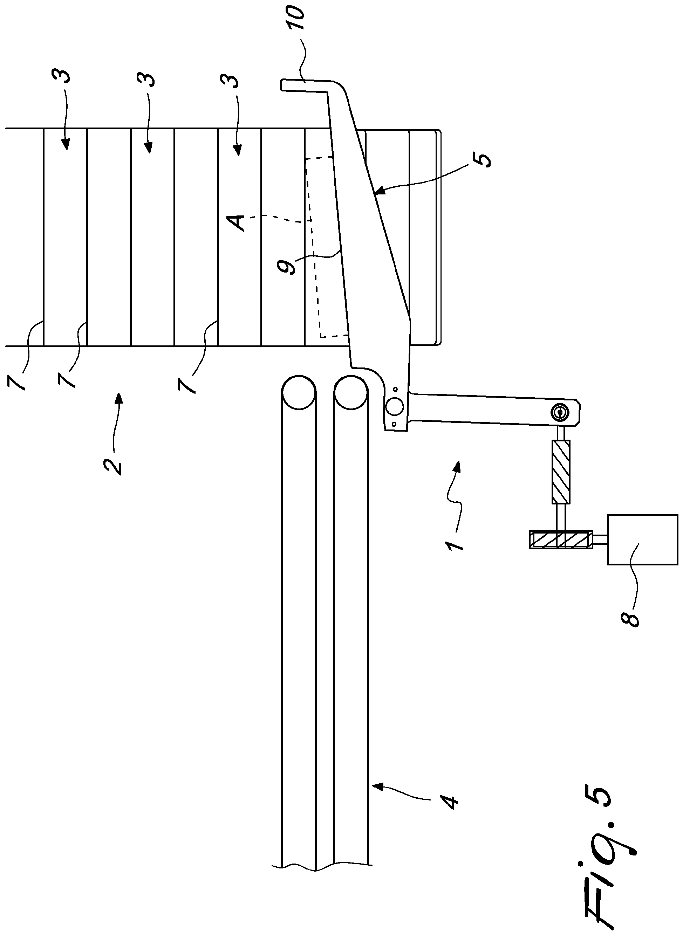

Figure 5 is a schematic top view of the assembly of Figure 1, showing a large product;

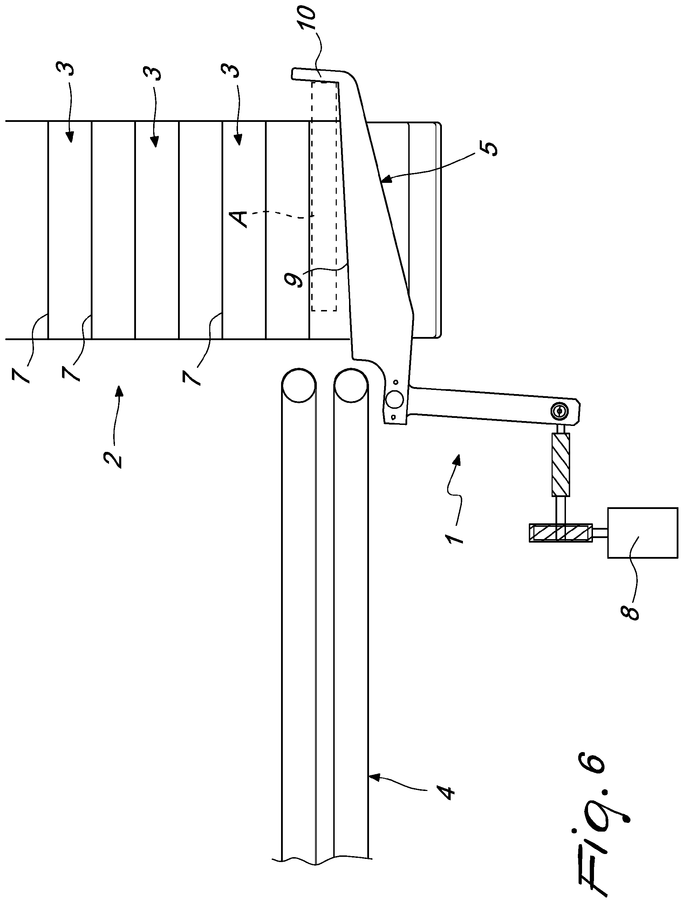

Figure 6 is a schematic top view of the assembly of Figure 1 in an extreme configuration.

With particular reference to the figures, the numeral 1 generally designates a halting assembly for hygienic and sanitary products A.

The assembly 1 is suitable to be installed in a station 2 for grouping products A within appropriate seats 3.

The products A arrive in sequence at the station 2, one behind the other, from a supply apparatus 4 arranged upstream.

The assembly 1 comprises at least one baffle 5, which faces and is proximate to the terminal edge 6 of the walls 7 that delimit the seats 3.

In practice, according to the constructive solution shown in the figure, the baffle 5 surmount the walls 7 and is therefore movable above the seats 3.

The baffle 5 is also applied in stations 2 in which the seats 3 are arranged differently (for example if the seats, instead of being open upward, as in the figure, are open with respect to a different direction): in any case, the baffle 5 faces the seats 3 and can move on a plane which faces and is proximate to the plane that contains the corresponding edges 6 of the walls 7.

The baffle 5 can move with respect to the seats 3 and its movement is generated by at least one respective actuator 8 which is controlled by a control and management unit.

The baffle 5 can move between a first configuration which is external to the seat 3, in which it does not interfere with the at least one product A that enters said seat, and a second configuration for maximum overlap on said seat 3, in which it locks the product A during first entry in the seat 3 with a halting surface 9 thereof.

In practice, the product A, sent into the seat 3 by the apparatus 4 with a predefined speed, can enter said seat 3 assuming different positions (starting from an only partial entry up to a condition of protrusion with respect to the back wall of the seat 3).

In order to ensure optimum packaging, each product A instead must be in the respective seat 3 always in the same position (i.e., halting of the product A within the seat 3 must always occur in a predefined position).

The baffle 5, by interfering with the stroke of the product A within the seat 3, halts it in the desired position, applying a gradual slowing by virtue of the friction applied by the face of the product A to its halting surface 9.

Said halting criterion can be applied in stations 2 which comprise seats 3 designed to accommodate a single product A at a time and in stations 2 in which each seat 3 can accommodate multiple products A arranged side by side (which constitute for example the bundle that will then be delivered to packaging).

If the seat 3 is designed to contain a single product A, the stroke of the product A is braked, and subsequently halted, by means of the clamping thereof between the wall 7 of the seat 3 and the surface 9 of the baffle 5.

If instead the seat 3 is designed to contain a plurality of products A, the stroke of each product A is braked, and subsequently stopped, by means of its clamping between the face of the product A that entered previously the seat 3 and the surface 9 of the baffle 5 (only the first product A that enters the seat 3 will be braked and subsequently stopped by means of the clamping thereof between the wall 7 of the seat 3 and the surface 9 of the baffle 5).

According to a particular embodiment of unquestionable interest in application, the baffle 5 can be advantageously pivoted, even indirectly, to the grouping station 2.

In this case, the actuator 8 is preset for the rotation of the baffle 5 with respect to the respective axis for pivoting to the station 2.

Greater rotations entail a greater interference of the baffle 5 with the seat 3 and therefore the halting of the product A proximate to the entry region of said seat 3; likewise, smaller rotations of the baffle 5 will entail less interference of the baffle 5 with the seat 3 and therefore the halting of the product A proximate to the terminal region of the seat 3.

According to a solution which is alternative to the one described previously, but is equally interesting in terms of embodiment, the baffle 5 can be validly able to slide with respect to the grouping station 2.

In this case, the actuator 8 is designed for the translation of the baffle 5 with respect to the station 2 according to a predefined stroke.

It is intuitive to identify that the more the baffle 5 is superimposed on the seat 3, the sooner the products A will be halted (i.e., will be closer to the entry region of said seat 3).

According to a further constructive solution which combines the advantages of both of the ones described previously, the baffle 5 can be conveniently pivoted to a carriage which can slide with respect to the grouping station 2.

In this particular version, the actuators 8 are two: a first actuator 8 is designed for the rotation of the baffle 5 with respect to the respective axis for pivoting to the carriage, while a second actuator 8 is designed for the translation of the carriage with respect to the station 2, according to a predefined stroke.

This second solution allows greater adjustment margins and allows to provide halting in ideal manners (it is possible to set the rule of motion for the halting of the product with extreme precision).

It is specified that the correct operation of the assembly 1 according to the invention depends on a correct placement of the baffle 5 as a function of the operating parameters of the station 2 and of the apparatus 4.

For example, the control and management unit must be able to select the position of the at least one baffle 5 with respect to the respective seat 3 as a function of the mass and speed of the hygienic and sanitary products A dispensed by the supply apparatus 4 arranged upstream.

In practice, products A having a large mass, which arrive with high speed, will have more kinetic energy and therefore halting by means of the baffle 5 will have to be performed so that the face of said product A that abuts against the surface 9 of the baffle 5 is subjected to intense friction during sliding (in order to slow it effectively): the baffle 5 must therefore interfere substantially with the seat 3.

Products A with a small mass, which arrive at low speed, instead have low kinetic energy and can be stopped easily even by keeping the baffle 5 in conditions of reduced interference with the seat 3.

Another possible constructive solution comprises sensors for detecting the position of each product A previously halted in the respective seat 3.

These sensors will be connected to the unit, for the feedback control of the arrangement of the baffle 5 actuated by the respective actuator 8 as a function of the position of the product A previously introduced in the respective seat 3 by the supply apparatus 4.

This will ensure optimum operation and optimum arrangement of the products A in the seats 3: indeed even if the environmental parameters (temperature, pressure, humidity) or the characteristics of the raw materials of which the products A are progressively made change during production, these changes do not affect their positioning, which is kept constant by virtue of the feedback adjustment of the baffle 5 designed to slow and halt each product A.

It is specified furthermore that the halting surface 9 of the baffle 5, on which one face of the product A slides during its slowing, has a shape which is preferably chosen among planar, with a curvilinear contour, with a sequence of mutually inclined planes, irregular, and combinations thereof.

The particular shape of the surface 9 allows to slow and halt the product A in the manner that is most suited for the specific product A being processed (depending on the consistency, dimensions and structural characteristics and the materials that constitute it, there may be specific requirements to be met).

With particular reference to a constructive solution of unquestionable interest in practice and in application, the baffle 5 can advantageously have an end tab 10 which is substantially transverse to the direction of advancement of the products A which forms a stroke limiting shoulder for said products A sent into the seat 3 by the supply apparatus 4.

In some cases, in fact, having a stroke limit for the product A ensures that said product assuredly cannot protrude with respect to an extreme arrangement which is defined indeed by the position of the end tab 10.

From a constructive standpoint, it is specified that the actuator 8 can favorably be chosen preferably from a gearmotor associated with a worm screw (in accordance with the constructive solution shown merely by way of nonlimiting example in the accompanying figures), an electric motor, optionally associated with respective motion transmission/reduction elements, a hydraulic motor, optionally associated with respective motion transmission/reduction elements, a pneumatic motor, optionally associated with respective motion transmission/reduction elements, and the like.

The choice to adopt an actuator 8 constituted by a gearmotor associated with a worm screw is advantageous since it excludes the possibility of retrograde motions, thus ensuring that the position assumed by the baffle 5 will not be modified as a consequence of the energy transferred thereto by the product A during impact therewith (although said impact must be performed gradually in order to perform progressive braking, as explained previously).

The method for the gradual halting of hygienic and sanitary products A that arrive from a supply apparatus 4 within at least one respective seat 3 of a station 2 provides for the execution of the following steps.

First of all, it is necessary to arrange the baffle 5 so that it is at least partially juxtaposed on the seat 3, in interference with the stroke of the products A that enter it.

Then one must adjust the position of the baffle 5 by means of a respective actuator 8 so that a face of said product A, entering said seat 3, slides on a halting surface 9 of the baffle 5 with a friction which is proportional to the extent of the interference of the baffle 5 with said seat 3.

Advantageously, the present invention solves the problems described above, proposing a halting assembly 1 for hygienic and sanitary products A that ensures the halting of the products A, within a respective drawer of a specific rack, more particularly within a respective seat 3 formed by consecutive walls 7, in a predefined neighborhood of the ideal position.

Validly, the assembly 1 according to the invention generates no deformation of the products A that enter the seats 3, since it does not halt them with an abutment surface against which they may collide while they are still in motion with a predefined speed, but slows them down during the sliding of one of their faces on the surface 9 of the baffle 5.

Positively, the assembly 1 according to the invention prevents the product A from rebounding backwards once it has reached the back wall of the respective drawer of a rack, i.e., once it has reached the back wall of the respective seat 3, since when each product A reaches the end surface 10 it has by then been slowed down sufficiently by friction to avoid any rebound.

Favorably, the halting assembly 1 for hygienic and sanitary products A is relatively simple to provide in practice and is substantially modest in cost: these characteristics make the station 1 according to the invention innovation of assured application.

The invention thus conceived is susceptible of numerous modifications and variations, all of which are within the scope of the appended claims; all the details may furthermore be replaced with other technically equivalent elements.

In the examples of embodiment shown, individual characteristics, given in relation to specific examples, may actually be interchanged with other different characteristics that exist in other examples of embodiment.

In practice, the materials used, as well as the dimensions, may be any according to the requirements and the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A halting assembly for hygienic and sanitary products (A) of the type suitable for installation in a station (2) for grouping products (A) within adapted seats (3), said products (A) arriving in sequence at said station (2), one after the other, from a supply apparatus (4) arranged upstream, **characterized in that** it comprises at least one baffle (5) that faces and is proximate to the terminal edge (6) of the walls (7) that delimit said seats (3) and is actuated by at least one respective actuator (8) controlled by a control and management unit, said baffle (5) being movable between a first configuration which is external to said seat (3), in which it does not interfere with the at least one product (A) in input thereto, and a second configuration for maximum overlap on said seat (3), in which it locks the product (A) during first entry in said seat (3) with a halting surface (9) thereof.

2. The assembly according to claim 1, **characterized in that** said baffle (5) is pivoted, even indirectly, to said grouping station (2), said actuator (8) being preset for the rotation of said baffle (5) with respect to the respective axis of pivoting to said station (2).

3. The assembly according to claim 1, **characterized in that** said baffle (5) can slide with respect to said grouping station (2), said actuator (8) being preset for the translation of said baffle (5) with respect to said station (2) according to a predefined stroke.

4. The assembly according to claim 1, **characterized in that** said baffle (5) is pivoted to a carriage which can slide with respect to said grouping station (2), said actuators (8) are two, a first actuator (8) being preset for the rotation of said baffle (5) with respect to the corresponding axis for pivoting to said carriage and a second actuator (8) being preset for the translation of said carriage with respect to said station (2), according to a predefined stroke.

5. The assembly according to claim 1, **characterized in that** said control and management unit selects the position of said at least one baffle (5) with respect to the respective seat (3) as a function of the mass and speed of the hygienic and sanitary products (A) dispensed by said supply apparatus (4) arranged upstream.

6. The assembly according to claim 1, **characterized in that** it comprises sensors for detecting the position of each said product (A) in the respective seat (3), said sensors being connected to said unit, for the control with feedback of the arrangement of said baffle (5) actuated by said actuator (8) as a function of the position of the product (A) previously introduced in the respective seat (3) by said supply apparatus (4).

7. The assembly according to one or more of the preceding claims, **characterized in that** said halting surface (9) of said baffle (5), on which one face of said product (A) slides, has a shape that is preferably chosen among planar, with a curvilinear contour, with a sequence of mutually inclined planes, irregular, and combinations thereof.

8. The assembly according to one or more of the preceding claims, **characterized in that** said baffle (5) has a substantially transverse end tab (10), which forms a stroke limiting shoulder for the products (A) sent into said seat (3) by said supply apparatus (4).

9. The assembly according to one or more of the preceding claims, **characterized in that** said actuator (8) is of the type preferably chosen among a gearmotor associated with a worm screw, an electric motor, optionally associated with respective motion transmission/reduction elements, a hydraulic motor, optionally associated with respective motion transmission/reduction elements, a pneumatic motor, optionally associated with respective motion transmission/reduction elements, and the like.

10. A method for the gradual halting of hygienic and sanitary products (A) originating from a supply apparatus (4) within at least one respective seat (3) of a grouping station (2), **characterized in that** it comprises the following steps:
- positioning a baffle (5) that is at least partially juxtaposed on said seat (3), in interference with the stroke of said products (A) in input thereto,
- adjusting the position of said baffle (5) by means of a respective actuator (8) so that one face of said product (A) entering said seat (3) slides on a halting surface (9) of said baffle (5) with a friction that is proportional to the extent of the interference of the baffle (5) with said seat (3).

## Patentansprüche

1. Eine Anhaltevorrichtung für Hygiene- und Sanitärprodukte (A) von der Art, die zum Einbau in eine Station (2) zur Gruppierung von Produkten (A) in passenden Sitzen (3) geeignet ist, wobei die Produkte (A) nacheinander von einer stromaufwärts angeordneten Zuführvorrichtung (4) an der Station (2) ankommen; **dadurch gekennzeichnet, dass** sie mindestens eine Ablenkplatte (5) umfasst, die der Abschlusskante (6) der Wände (7), die die Sitze (3) begrenzen, zugewandt ist und an sie angrenzt und von mindestens einem entsprechenden Antriebselement (8) angetrieben ist, das von einer Steuerungs- und Verwaltungseinheit gesteuert ist; wobei die Ablenkplatte (5) beweglich ist zwischen einer ersten Anordnung, die außerhalb des Sitzes (3) liegt und in der sie das mindestens eine Produkt (A), das dort eingeht, nicht behindert, und einer zweiten Anordnung zur maximalen Überlagerung an dem Sitz (3), in welcher sie das Produkt (A) während des ersten Eintritts in den Sitz (3) mit einer Anhalteoberfläche (9) derselben blockiert.

2. Die Vorrichtung gemäß Anspruch 1, **dadurch gekennzeichnet, dass** die Ablenkplatte (5), auch indirekt, gelenkig mit der Gruppierstation (2) verbunden ist, wobei das Antriebselement (8) für die Drehung der Ablenkplatte (5) mit Bezug auf die entsprechende Schwenkachse zu der Station (2) voreingestellt ist.

3. Die Vorrichtung gemäß Anspruch 1, **dadurch gekennzeichnet, dass** die Ablenkplatte (5) im Verhältnis zu der Gruppierungsstation (2) gleiten kann, wobei das Antriebselement (8) für die Translation der Ablenkplatte (5) im Verhältnis zu der Station (2) mit einem vordefinierten Hub voreingestellt ist.

4. Die Vorrichtung gemäß Anspruch 1, **dadurch gekennzeichnet, dass** die Ablenkplatte (5) gelenkig mit einem Schlitten verbunden ist, der im Verhältnis zu der Gruppierungsstation (2) gleiten kann; die Antriebselemente (8) sind zwei an der Zahl, wobei ein erstes Antriebselement (8) voreingestellt ist für die Drehung der Ablenkplatte (5) mit Bezug auf die entsprechende Schwenkachse zu dem Schlitten und ein zweites Antriebselement (8) voreingestellt ist für die Translationsbewegung des Schlittens mit Bezug auf die Station (2) mit einem vordefinierten Hub.

5. Die Vorrichtung gemäß Anspruch 1, **dadurch gekennzeichnet, dass** die Steuerungs- und Verwaltungseinheit die Position der mindestens einen Ablenkplatte (5) im Verhältnis zu dem entsprechenden Sitz (3) in Abhängigkeit von der Masse und Geschwindigkeit der Hygiene- und Sanitärprodukte (A) auswählt, die von der stromaufwärts angeordneten Zuführvorrichtung (4) abgegeben werden.

6. Die Vorrichtung gemäß Anspruch 1, **dadurch gekennzeichnet, dass** sie Sensoren zur Erfassung der Position jedes Produkts (A) in dem jeweiligen Sitz (3) umfasst, wobei die Sensoren mit der Einheit verbunden sind zur Steuerung, mit Rückkopplung, der Anordnung der Ablenkplatte (5), die von dem Antriebselement (8) angetrieben wird, in Abhängigkeit von der Position des Produkts (A), das zuvor von der Zuführvorrichtung (4) in den jeweiligen Sitz (3) eingesetzt wurde.

7. Die Vorrichtung gemäß einem oder mehreren der obigen Ansprüche, **dadurch gekennzeichnet, dass** die Anhalteoberfläche (9) der Ablenkplatte (5), auf der eine Fläche des Produkts (A) gleitet, eine Form hat, die vorzugsweise gewählt ist aus planar, mit einer krummlinigen Kontur, mit einer Folge zueinander schräger Ebenen, unregelmäßig und Kombinationen davon.

8. Die Vorrichtung gemäß einem oder mehreren der obigen Ansprüche, **dadurch gekennzeichnet, dass** die Ablenkplatte (5) eine im Wesentlichen transversale Endnase (10) hat, die eine Hubbegrenzungsschulter für die Produkte (A) bildet, welche von der Zuführvorrichtung (4) in den Sitz (3) transportiert werden.

9. Die Vorrichtung gemäß einem oder mehreren der obigen Ansprüche, **dadurch gekennzeichnet, dass** das Antriebselement (8) von der Art ist, die vorzugsweise gewählt ist aus einem mit einer Schneckenschraube verbundenen Getriebemotor, einem Elektromotor, wahlweise verbunden mit entsprechenden Bewegungsübertragungs-/Reduktionselementen, einem hydraulischen Motor, wahlweise verbunden mit entsprechenden Bewegungsübertragungs-/ Reduktionselementen, einem pneumatischen Motor, wahlweise verbunden mit entsprechenden Bewegungsübertragungs-/ Reduktionselementen, und dergleichen.

10. Ein Verfahren zum allmählichen Anhalten von Hygiene- und Sanitärprodukten (A), die von einer Zuführvorrichtung (4) stammen, in mindestens einem entsprechenden Sitz (3) einer Gruppierungsstation (2), **dadurch gekennzeichnet, dass** es folgende Schritte umfasst:
- das Positionieren einer Ablenkplatte (5), die zumindest teilweise auf den Sitz (3) gelegt ist, in Interferenz mit dem Hub der Produkte (A), die dort eingehen,
- das Anpassen der Position der Ablenkplatte (5) mit Hilfe eines entsprechenden Antriebselements (8), so dass eine Fläche des Produkts (A), das in den Sitz (3) eintritt, auf einer Anhalteoberfläche (9) der Ablenkplatte (5) mit einer Reibung gleitet, die proportional zum Grad der Interferenz der Ablenkplatte (5) mit dem Sitz (3) ist.

## Revendications

1. Ensemble d'arrêt pour des produits hygiéniques et sanitaires (A) du type adapté pour une installation dans une station (2) destinée à regrouper des produits (A) à l'intérieur de logements (3) adaptés, lesdits produits (A) arrivant en séquence sur ladite station (2), les uns après les autres, à partir d'un appareil d'alimentation (4) agencé en amont, **caractérisé en ce qu'**il comporte au moins un déflecteur (5) qui fait face au bord terminal (6) des parois (7) délimitant lesdits logements (3) et qui est près de celui-ci, et est actionné au moyen d'un actionneur (8) respectif commandé par une unité de commande et de gestion, ledit déflecteur (5) étant mobile entre une première configuration qui est extérieure audit logement (3), dans laquelle il n'interfère pas avec le au moins un produit (A) en entrée de celui-ci, et une seconde configuration de chevauchement maximal sur ledit logement (3), dans laquelle il bloque le produit (A) pendant une première entrée dans ledit logement (3) avec une surface d'arrêt (9) de celui-ci.

2. Ensemble selon la revendication 1, **caractérisé en ce que** ledit déflecteur (5) pivote, même indirectement, sur ladite station de regroupement (2), ledit actionneur (8) étant préréglé pour la rotation dudit déflecteur (5) par rapport à l'axe de pivotement respectif sur ladite station (2).

3. Ensemble selon la revendication 1, **caractérisé en ce que** ledit déflecteur (5) peut coulisser par rapport à ladite station de regroupement (2), ledit actionneur (8) étant préréglé pour la translation dudit déflecteur (5) par rapport à ladite station (2) en fonction d'une course prédéfinie.

4. Ensemble selon la revendication 1, **caractérisé en ce que** ledit déflecteur (5) pivote jusqu'à un chariot qui peut coulisser par rapport à ladite station de regroupement (2), lesdits actionneurs (8) sont au nombre de deux, un premier actionneur (8) étant préréglé pour la rotation dudit déflecteur (5) par rapport à l'axe correspondant pour pivoter sur ledit chariot et un second actionneur (8) étant préréglé pour la translation dudit chariot par rapport à ladite station (2), en fonction d'une course prédéfinie.

5. Ensemble selon la revendication 1, **caractérisé en ce que** ladite unité de commande et de gestion sélectionne la position dudit au moins un déflecteur (5) par rapport au logement (3) respectif en fonction de la masse et de la vitesse des produits hygiéniques et sanitaires (A) distribués par ledit appareil d'alimentation (4) agencé en amont.

6. Ensemble selon la revendication 1, **caractérisé en ce qu'**il comporte des capteurs pour détecter la position de chaque dit produit (A) dans le logement (3) respectif, lesdits capteurs étant reliés à ladite unité, pour la commande avec asservissement de l'agencement dudit déflecteur (5) actionné par ledit actionneur (8) en fonction de la position du produit (A) précédemment introduit dans le logement (3) respectif par ledit appareil d'alimentation (4).

7. Ensemble selon une ou plusieurs des revendications précédentes, **caractérisé en ce que** ladite surface d'arrêt (9) dudit déflecteur (5), sur laquelle une face dudit produit (A) coulisse, a une forme qui est de préférence choisie parmi plane, avec un contour curviligne, avec une séquence de plans mutuellement inclinés, irrégulière, et des combinaisons de celles-ci.

8. Ensemble selon une ou plusieurs des revendications précédentes, **caractérisé en ce que** ledit déflecteur (5) a une languette d'extrémité sensiblement transversale (10), qui forme un épaulement de limitation de course pour les produits (A) envoyés dans ledit logement (3) par ledit appareil d'alimentation (4).

9. Ensemble selon une ou plusieurs des revendications précédentes, **caractérisé en ce que** ledit actionneur (8) est du type de préférence choisi parmi un moteur à engrenages associé à une vis sans fin, un moteur électrique, facultativement associé à des éléments de transmission/réduction de mouvement respectifs, un moteur hydraulique, facultativement associé à des éléments de transmission/réduction de mouvement respectifs, un moteur pneumatique, facultativement associé à des éléments de transmission/réduction de mouvement respectifs, et analogue.

10. Procédé pour l'arrêt graduel de produits hygiéniques et sanitaires (A) provenant d'un appareil d'alimentation (4) à l'intérieur d'au moins un logement (3) respectif d'une station de regroupement (2), **caractérisé en ce qu'**il comporte les étapes suivantes consistant à :
- positionner un déflecteur (5) qui est au moins partiellement juxtaposé audit logement (3), en interférence avec la course desdits produits (A) en entrée de celui-ci,
- régler la position dudit déflecteur (5) au moyen d'un actionneur (8) respectif de sorte qu'une face dudit produit (A) entrant dans ledit logement (3) coulisse sur une surface d'arrêt (9) dudit déflecteur (5) avec un frottement qui est proportionnel à l'ampleur de l'interférence du déflecteur (5) avec ledit logement (3).
